## Europäisches Patentamt
## European Patent Office
## Office européen des brevets

(11) Veröffentlichungsnummer: **0 076 872**
**A1**

(12) **EUROPÄISCHE PATENTANMELDUNG**

(21) Anmeldenummer: **81108223.9**

(22) Anmeldetag: **12.10.81**

(51) Int. Cl.³: **A 61 M 5/00, A 61 M 5/31**

(43) Veröffentlichungstag der Anmeldung: **20.04.83**
Patentblatt **83/16**

(71) Anmelder: **Petz Electro, Friesenstrasse 791, CH-3185 Schmitten (CH)**

(72) Erfinder: **Petz, Günter, Flachslander Strasse 8, D-8500 Nürnberg (DE)**

(84) Benannte Vertragsstaaten: **AT BE CH DE FR GB IT LI LU NL SE**

(74) Vertreter: **Göbel, Matthias, Dipl.-Ing., Pruppacher Hauptstrasse 5-7, D-8501 Pyrbaum-Pruppach (DE)**

(54) **Diabetiker-Set.**

(57) Bei einem Diabetiker-Set mit einem durch einen Aufnahmeteil und einem abziehbaren kappenartigen Deckel gebildeten Aufnahmegehäuse für in Halterungen des Aufnahmeteils lösbar fixierte Insulinflaschen und Injektionsspritzen, bei dem der Aufnahmeteil oder der Deckel Alkoholtücher aufnehmen, ist zum Zwecke der Vereinfachung des baulichen Aufwands und der Erleichterung der Handhabung des Diabetiker-Sets der Aufnahmeteil durch einen einseitig offenen etuiförmigen Hohlkörper (1) und einen in diesen über eine Teillänge fest angeordneten kastenförmigen Einsatz (2) gebildet, der die Insulinflaschen (4) und die Injektionsspritzen (5) lösbar fest haltert sowie den Deckel (3) schieb- und klappbar hält, während axial unterhalb des Einsatzes (2) im Hohlkörper (1) eine über eine verschließbare Seitenwandöffnung (16) zugängliche Kammer (15) Alkoholtücher aufnimmt.

DIPL.-ING. **M. GÖBEL**

**PATENTANWALT**

3501 PYRBAUM-PRUPPACH

RUPPACHER HAUPTSTRASSE 57
TELEFON 0 9180 / 675
TELEGRAMM GOEPATENT PYRBA IM
TELEX 624407 GOEPA

BANKKONTEN
VOLKSBANK NÜRNBERG 45 233 BLZ 760 900 00
COMMERZBANK NÜRNBERG 8 300 907 BLZ 760 400 61

0076872

- 1 -

Petz Electro, CH 3185 Schmitten

Diabetiker-Set

Die Erfindung betrifft einen Diabetiker-Set mit einem durch einen Aufnahmeteil und einem abziehbaren kappenartigen Deckel gebildeten Aufnahmegehäuse für in Halterungen des Aufnahmeteils lösbar fixierte Insulinflaschen und Injektionsspritzen, bei dem der Aufnahmeteil oder der Deckel Alkoholtücher aufnehmen.

Es sind bereits durch einen Kasten mit Klappdeckel gebildete Diabetiker-Set bekannt, die die Insulinflaschen und Injektionsspritzen in Klemmhalterungen aufnehmen. Diese Diabetiker-Sets sind unhandlich und zu ihrer Benutzung in unhygienischer Weise jeweils auf Ablageflächen aufzustellen. Darüber hinaus ist ein Diabetiker-Set (Deutsche Patentanmeldung P 30 29 226.3) mit einem etuiartigen oder schalenartigen Aufnahmegehäuse für die Insulinflaschen und Injektionsspritzen und einem abziehbaren Deckel vorgeschlagen worden. Die Gehäuse dieser Diabetiker-Set bedürfen jedoch einen großen fertigungstechnischen Aufwand, insbesondere für die Ausbildung der Klemmhalterungen für die Insulinflaschen und Injektionsspritzen. Außerdem führt der abnehmbare Deckel zu einer ungünstigen Beeinträchtigung der Handhabung dieser Diabetiker-Sets bei der Entnahme der Injektionsspritzen und Insulins.

Ausgehend von einem Diabetiker-Set der letztgenannten Art ist es Aufgabe der Erfindung Maßnahmen zur Vereinfachung des baulichen Aufwands des Aufnahmegehäuses und zur Erleichterung der Handhabung dieser Diabetiker-Sets zu schaffen.

Der Erfindung gemäß ist hierzu vorgesehen, daß der Aufnahmeteil für die Insulinflaschen und Injektionsspritzen durch einen einseitig offenen etuiförmigen Hohlkörper und einem in diesem über eine Teillänge fest angeordneten kastenförmigen Einsatz gebildet ist, der die Insulinflaschen und die Injektionsspritzen lösbar fest haltert sowie den Deckel schieb- und klappbar hält und daß axial unterhalb des Einsatzes im Hohlkörper eine über eine verschließbare Seitenwandöffnung zugängliche Kammer für die Aufnahme von Alkoholtüchern vorgesehen ist. Durch die Anordnung von zwei einander umfassenden Gehäuseteilen für den Aufnahmeteil ist ein einfach herstellbares Aufnahmegehäuse geschaffen und durch die beweglich feste Verbindung von Aufnahmegehäuse und Deckel ist die Voraussetzung zur Halterung des Diabetiker-Set mit einer einzigen Hand des Benutzers gegeben. Hierdurch erhält der Benutzer Gelegenheit mit der anderen freien Hand die Insulinspritzvorgänge unbehindert durchzuführen.

In Ausgestaltung des Diabetiker-Sets kann der kastenförmige Einsatz in mindestens einer Seitenwand Ausnehmungen und in diesen bogenförmig nach innen und zurück gebogene federnde Haltelappen für die Insulinflaschen aufweisen. Zweckmäßig sind in zwei einander gegenüberliegenden Seitenwänden Ausnehmungen mit Haltelappen vorgesehen. Die Haltelappen nehmen die Insulinflaschen zwischen sich klemmend auf, wobei eine Fixierung von Insulinflaschen mit verschieden großen Durchmessergrößen erfolgen kann bzw. ein Ausgleich von Durchmessertoleranzen bewirkt wird. Zweckmäßig sind die Haltelappen mit zu den Ausnehmungen wesentlich geringerer Breite und Länge ausgebildet, wodurch sich neben und ob-erhalb der Haltelappen streifenförmige Durchblicköffnungen ergeben, die als Fensteröffnungen das Erkennen der Insulinflaschen und deren Inhalt ermöglichen. Der Benutzer braucht demgemäß die Insulinflaschen zur Kontrolle des Füllgrades derselben nicht aus ihrer Halterung herausnehmen. Darüber hinaus ist vorgesehen im Einsatzkörper in mindestens einer Seitenwand ob-erhalb der Ausnehmungen noch zusätzliche Durchblicköffnungen vorzusehen, über die auch das obere Ende der Insulinflaschen erkennbar ist.

Eine Verbesserung der Halterung der Insulinflaschen läßt sich noch dadurch herbeiführen, daß in der oberen Seitenwand des Einsatzes Durchstecköffnungen für die Insulinflaschen vorgesehen sind, deren Begrenzungs- bzw. Randflächen als Stützflächen für den Hals bzw. den Kopf der Insulinflaschen dienen.

Um bei einer sicheren Halterung eine leichte Entnahme der Injektionsspritzen vom Einsatzkörper zu gewährleisten, ist in weiterer Ausgestaltung des Diabetiker-Sets vorgesehen, den Einsatzkörper mit federnd elastisch abspreizbaren Anformungen oder Anbiegungen zu versehen, an bzw. zwischen die die Injektionsspritzen abnehmbar festgelegt sind. Die Injektionsspritzen können mit seitlichen Verlängerungen zwischen den Anformungen oder Anbiegungen eingreifen und gehalten sein. Eine Entnahme ist durch einfaches Zurückbiegen der Anformungen möglich, wobei das Zurückbiegen beliebig mit dem Daumen der freien Hand des Benutzers bzw. selbsttätig durch eine quer zur Abnahmekraft wirkende Komponente derselben erfolgt.

Zum Einbringen oder Entnehmen von Alkoholtüchern kann die Seitenwandöffnung der Kammer durch einen am Hohlkörper verschließbar angeordneten Wandabschnitt übergriffen sein. Es besteht auch die Möglichkeit, den Wandabschnitt verschwenkbar am Hohlkörper angreifen zu lassen, so daß durch einen einfachen Verschiebe- oder Verschwenkvorgang die Aufnahmekammer für die Alkoholtücher geöffnet oder geschlossen werden kann.

Damit der Deckel einen sicheren Schutz für die Insulinflaschen und Injektionsspritzen auch beim Transport des Diabetiker-Sets ergibt, ist weiter vorgesehen, den Deckel mittels Rastenkörper am Einsatz und/oder Hohlkörper freigebbar festzulegen. Als Rastenkörper können streifenförmige Anformungen am Deckel sowie im Einsatz und/oder Hohlkörper eingeformte Rastnuten für die Aufnahme der Anformungen dienen.

Es versteht sich, daß der Diabetiker-Set mit beliebiger Größe und aus beliebigen Werkstoffen herstellbar ist. Zum Zwecke eines sicheren Erfassens können die Gehäuseteile im Bereich der Oberfläche mit Grifflächen versehen

sein. Als Griffflächen sind Aufrauhungen oder Narbungen denkbar. Ferner besteht die Möglichkeit die Gehäuseteile aus einem Hartkunststoff oder einem flexiblen nachgiebigen Kunststoff auszubilden.

Wie die Erfindung ausgeführt sein kann, zeigen mit den für diese wesentlichen Merkmalen, die in der Zeichnung dargestellten Ausführungsbeispiele. Es bedeuten:

Fig. 1 einen Diabetiker-Set im Längsschnitt,
Fig. 2 einen Schnitt nach der Linie II-II der Fig. 1,
Fig. 3 einen Schnitt nach der Linie III-III der Fig. 1,
Fig. 4 einen Diabetiker-Set in Seitenansicht mit aufgeklapptem Deckel,
Fig. 5 einen Diabetiker-Set geschlossen in Seitenansicht,
Fig. 6 einen Diabetiker-Set gemäß einer abgewandelten Ausführungsform im Längsschnitt,
Fig. 7 einen Schnitt nach der Linie VII-VII der Fig. 6,
Fig. 8 einen Schnitt nach der Linie VIII-VIII der Fig. 6,
Fig. 9 einen Diabetiker-Set in Seitenansicht mit zurückgeklapptem Deckel und
Fig. 10 einen Diabetiker-Set in Seitenansicht mit einer geöffneten Aufnahmekammer für Alkoholtücher.

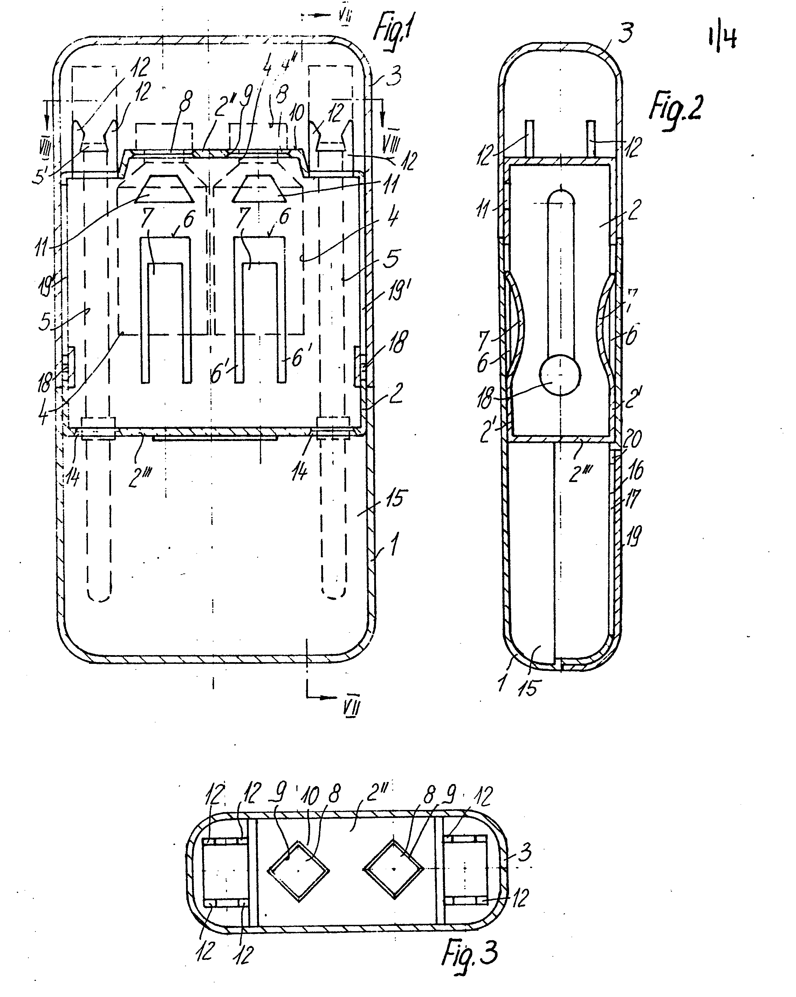

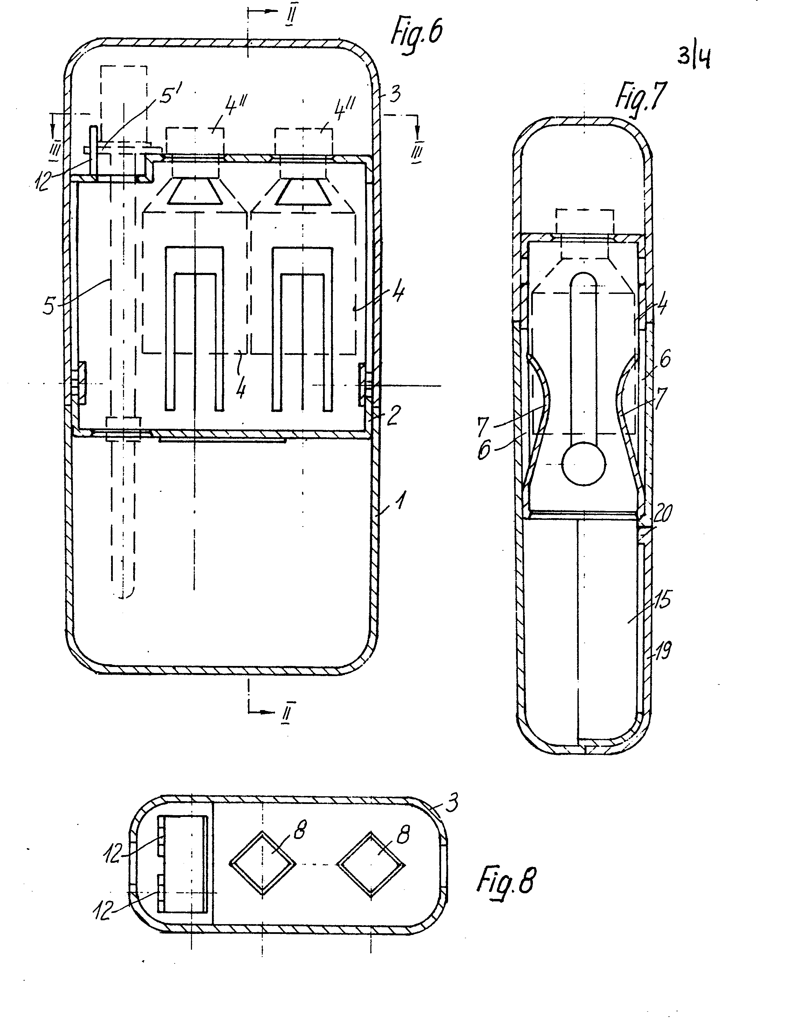

Beim Diabetiker-Set nimmt ein etuiförmiger Hohlkörper 1 über eine Teillänge einen kastenförmigen Einsatz 2 auf, der mit dem Hohlkörper fest verbunden ist. Der Hohlkörper 1 und der kastenförmige Einsatz 2 bilden zusammen mit einem am Einsatz 2 verschieb- und schwenkbar gehalterten Deckel 3 das Aufnahmegehäuse für zwei Insulinflaschen 4 und zwei Injektionsspritzen 5. Beim Ausführungsbeispiel weist der Einsatz 2 in den beiden einander gegenüberliegenden Seitenwänden 2' Ausnehmungen 6 auf, die nach innen und zurück gebogene federnde Haltelappen 7 aufnehmen (Fig. 2, 7) Zwischen je zwei einander gegenüberliegenden Haltelappen 7 sind die Insulinflaschen 4 klemmend festgelegt. Die Ausnehmungsabschnitte 6' neben oder oberhalb der Haltelappen 7 dienen als Fensteröffnungen zur Betrachtung der Insulinflaschen 4 und zum Erkennen des Füllgrades derselben. Im Bereich der oberen Seitenwand 2" des Einsatzes 2 durchgreifen die Insulinflaschen 4 Durchstecköffnungen 8, deren Begrenzungsflächen 9 den Hals 4' der Insulinflaschen 4 bzw. deren Randflächen 10 den Kopf 4" der Insulinflaschen 4 stützen. Die Durchstecköffnungen 8 sind in ihrer Querschnittsgröße so bemessen, daß auch ein unbeabsichtigtes Herausfallen der Insulinflaschen 4 aus dem Einsatz 2 verhindert ist. Im Abstand oberhalb der Ausnehmungen 6 sind in den beiden Seitenwänden 2' des Einsatzes 2 weitere Durchblicköffnungen 11 zum Erkennen der Insulinflaschen 4 vorgesehen.

Beim Diabetiker-Set der Figuren 1 bis 4 weist die obere Seitenwand 2" zu beiden Seiten der Insulinflaschen 4 federnd elastisch abbiegbare Anformungen 12 auf, zwischen die die Injektionsspritzen 5, insbesondere mit seitlichen Verlängerungen 5' eingreifen. Die gegenüber den Insulinflaschen 4 wesentlich längeren Injektionsspritzen 5 durchragen außerdem Öffnungen 14 im Boden 2''' des Einsatzes 2 und tauchen in einer Kammer 15 unterhalb des Einsatzes 2 ein. Die Kammer 15 dient der Aufnahme von nicht näher dargestellten Alkoholtüchern. Sie ist durch eine Öffnung 16 in einer Seitenwand 1'' zugänglich, wobei die Öffnung 16 durch einen verschieblichen Wandabschnitt 19 verschließbar ist, der beim Ausführungsbeispiel mittels Anformungen 20 in Nuten 17 des Hohlkörpers 1 geführt ist.

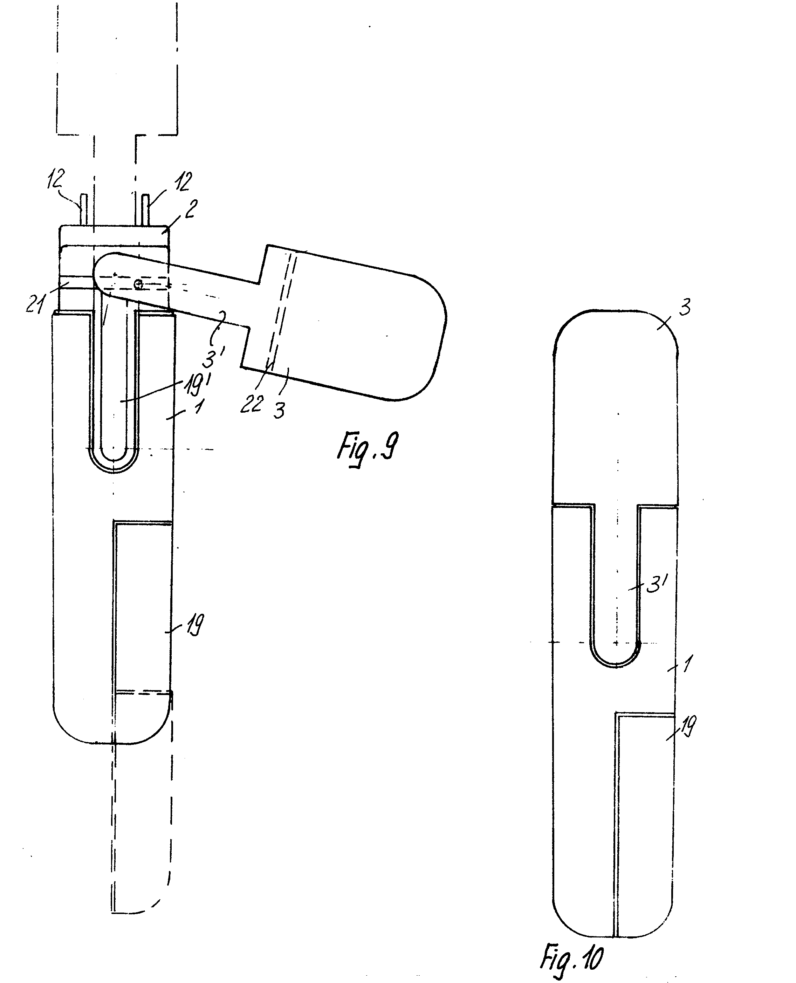

Abweichend sind beim Diabetiker-Set der Figuren 6 bis 9 die die Injektionsspritzen haltenden beiden Anformungen 12 auf einer Seite der Insulinflaschen 4 verlegt.

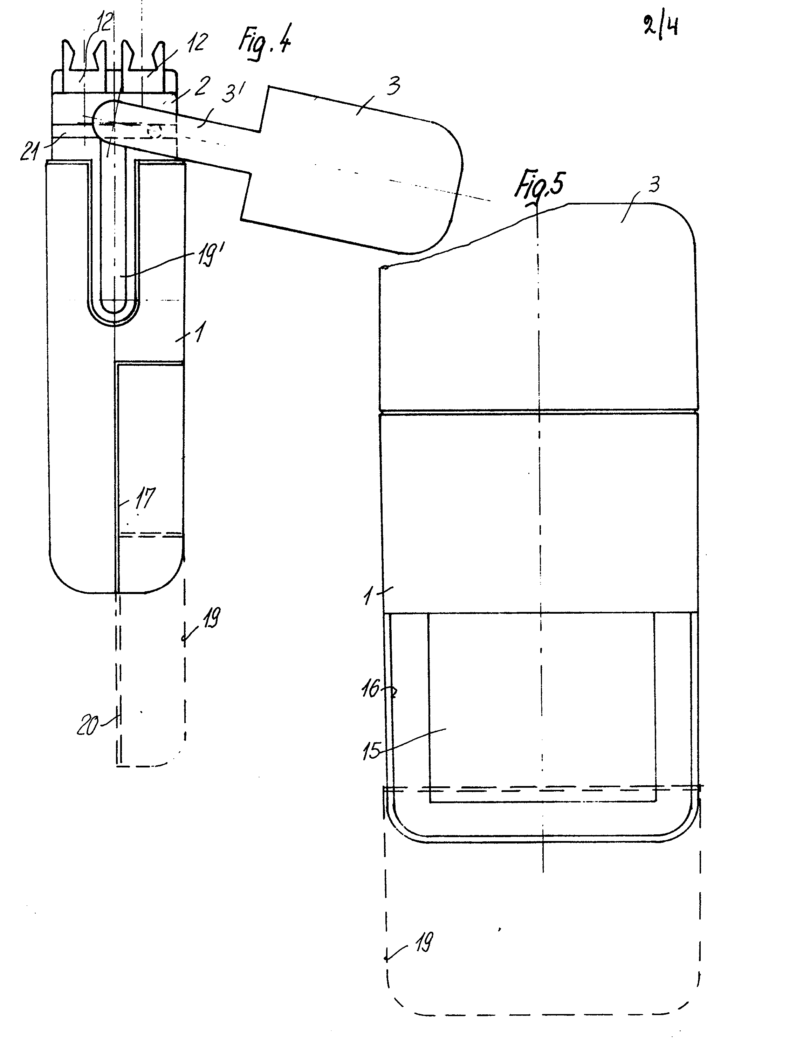

Zur Benutzung des Diabetiker-Sets ist der etuiförmige Hohlkörper 1 mitsamt dem Einsatz 2 durch eine Hand des Benutzers zu halten. Mit der anderen Hand kann der Benutzer den Deckel 3 nach oben abschieben und durch Umklappen in die Offenstellung der Figur 4 bringen. Der Deckel 3 verbleibt hierbei mit laschenförmigen Ansätzen 3' und Stiften 18 in Nuten 19' des Einsatzes 2. Danach kann der Benutzer mit der zuvor den Deckel 3 betätigenden Hand eine Injektionsspritze 5 aus den Anformungen 12 entnehmen und nach dem Einstechen in eine der Insulinflaschen 4, Insulin aufziehen. Nach der Benutzung kann die Injektionsspritze 5 in die Anformungen zurückgebracht werden. Dem Benutzer wird ermöglicht über die Ausnehmungsabschnitte 6' und den Durchblicköffnungen 11 die Insulinflaschen 4 und deren Füllgrad einzusehen. Durch Erfassen des Deckels 3, Aufwärtsschwenken mit nachfolgendem Aufschieben desselben auf den Einsatz 2 ist der Diabetiker-Set verschießbar. (Fig. 5 und 10).

Zur Unterbringung von Alkoholtüchern in die Kammer 15, ist der Wandabschnitt 19 zunächst nach unten in die gestrichelt gezeichnete Stellung abzuschieben (Fig. 4, 5 und 9). Über die freie Öffnung 16 können nunmehr die Alkoholtücher in die Kammer 15 eingebracht werden. Durch Aufwärtsschieben des Wandabschnitts 19 ist die Kammer 15 verschließbar (Fig. 2, 7). Die Entnahme von Alkoholtüchern erfolgt in ebensolcher Weise.

0076872

Die erfindungsgemäße Ausbildung des Diabetiker-Sets erlaubt die Halterung desselben mit einer Hand, während die Betätigung und die Benutzung des Inhalts durch die freie andere Hand des Benutzers erfolgen kann.

Patentansprüche

1. Diabetiker-Set mit einem durch einen Aufnahmeteil und einem abziehbaren kappenartigen Deckel gebildeten Aufnahmegehäuse für in Halterungen des Aufnahmeteils lösbar fixierte Insulinflaschen und Injektionsspritzen, bei dem der Aufnahmeteil oder der Deckel Alkoholtücher aufnehmen, dadurch gekennzeichnet, daß der Aufnahmeteil durch einen einseitig offenen etuiförmigen Hohlkörper (1) und einen in diesen über eine Teillänge fest angeordneten kastenförmigen Einsatz (2) gebildet ist, der die Insulinflaschen (4) und die Injektionsspritzen (5) lösbar fest haltert sowie den Deckel (3) schieb- und klappbar hält und daß axial unterhalb des Einsatzes (2) im Hohlkörper (1) eine über eine verschließbare Seitenwandöffnung (16) zugängliche Kammer (15) für die Aufnahme von Alkoholtüchern vorgesehen ist.

2. Diabetiker-Set nach Anspruch 1, dadurch gekennzeichnet, daß der Einsatz (2) in mindestens einer Seitenwand (2') Ausnehmungen (6) und in diesen bogenförmig nach innen und zurück gebogene federnde Haltelappen (7) für die Insulinflaschen (4) aufweist.

3. Diabetiker-Set nach Anspruch 2, dadurch gekennzeichnet, daß die Haltelappen (7) mit zu den Ausnehmungen (6) wesentlich geringerer Breite und Länge ausgebildet sind.

4. Diabetiker-Set nach Anspruch 1, dadurch gekennzeichnet, daß in der oberen Seitenwand (2'') des Einsatzes (2) Durchstecköffnungen (8) für die Insulinflaschen (4) angeordnet sind, deren Begrenzungs- (9) bzw. Randflächen (10) als Stützflächen für den Hals (4') bzw. den Kopf (4'') der Insulinflaschen (4) dienen.

5. Diabetiker-Set nach Anspruch 1, dadurch gekennzeichnet, daß der Einsatz (2) in mindestens einer Seitenwand im Abstand oberhalb der Ausnehmungen (6) Durchblicköffnungen (11) aufweist.

6. Diabetiker-Set nach Anspruch 1, dadurch gekennzeichnet, daß der Einsatz (2) federnd elastisch abspreizbare Anformungen (12) oder Anbiegungen aufweist, an bzw. zwischen die die Injektionsspritzen (5) freigebbar festgelegt sind.

7. Diabetiker-Set nach Anspruch 1, dadurch gekennzeichnet, daß die Öffnung (16) des Hohlkörpers (1) durch einen am Hohlkörper (1) verschiebbar angeordneten Wandabschnitt (19) verschließbar ist.

8. Diabetiker-Set nach Anspruch 1, dadurch gekennzeichnet, daß die Öffnung (16) des Hohlkörpers (1) durch einen am Hohlkörper (1) verschwenkbar angeordneten Wandabschnitt (19) verschließbar ist.

9. Diabetiker-Set nach Anspruch 1, dadurch gekennzeichnet, daß der Deckel (3) durch laschenartige Ansätze (3') mit Stiften (18) in Nuten (19') des Einsatzes (2) verschieb- und schwenkbar gehalten ist und mittels durch Nuten (21) gebildete und streifenförmige Anformungen (20)/Rastenkörper am Einsatz (2) freigebbar fixiert ist.

10. Diabetiker-Set nach Anspruch 7, dadurch gekennzeichnet, daß der Wandabschnitt (19) mittels streifenförmigen Anformungen (20) und am Hohlkörper (1) ausgebildete Rastnuten (17) am Hohlkörper (1) geführt ist.

1/4

*Fig.1*

*Fig.2*

*Fig.3*

Fig.4

Fig.5

Fig.6

Fig.7

Fig.8

12    12    2

21

19'    3'

19'

22    3

1

19

*Fig. 9*

3

3'

1

19

*Fig. 10*

Europäisches
Patentamt

EUROPÄISCHER RECHERCHENBERICHT

0076872

Nummer der Anmeldung

EP 81 10 8223

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | CH-A- 214 738 (PIATTI) <br><br> * Seite 2, Zeilen 30-66; Figuren 1-5 * <br><br> --- | | A 61 M 5/00 <br> A 61 M 5/31 |
| A | FR-A-2 050 952 (C.E.A.) <br><br> --- | | |
| A | GB-A-2 069 976 (PETERS) <br><br> --- | | |
| A | FR-A-1 548 574 (MICRO-MEGA) <br><br> --- | | |
| A | DE-A-2 039 341 (SCHMITZ) <br><br> ----- | | |

RECHERCHIERTE SACHGEBIETE (Int. Cl. ³)

A 45 C
A 61 B
A 61 C
A 61 M
B 65 D

Der vorliegende Recherchenbericht wurde für alle Patentansprüche erstellt.

| Recherchenort | Abschlußdatum der Recherche | Prüfer |
|---|---|---|
| DEN HAAG | 10-06-1982 | DURAND-SMET J.E.J.S. |